# EUROPEAN PATENT APPLICATION

(11) **EP 2 433 638 A2**
(43) Date of publication of application: **28.03.2012**
(21) Application number: 10777956.3
(22) Date of filing: 20.05.2010
(51) Int. Cl.: A61K 36/284, A61P 1/00, A23L 1/30, A61P 1/12

(54) **COMPOSITION FOR PREVENTING OR TREATING IRRITABLE BOWEL SYNDROME**

(30) Priority: 22.05.2009 KR 20090045167
(71) Applicant: SK Chemicals, Co., Ltd., Suwon-si, Gyeonggi-do 440-745 (KR)
(72) Inventor: SUN, Won Suck, Seoul 120-090 (KR); KIM, Taek-Su, Suwon-si Gyeonggi-do 440-300 (KR); KIM, Woong Sik, Suwon-si Gyeonggi-do 443-370 (KR); KUM, Do-Seung, Yongin-si Gyeonggi-do 448-971 (KR); RYU, Keun-Ho, Seoul 151-774 (KR); RHEE, Hae-In, Yongin-si Gyeonggi-do 448-918 (KR); JEON, Hyo Jin, Yongin-si Gyeonggi-do 448-130 (KR); MIN, Dong Sun, Seongnam-si Gyeonggi-do 463-731 (KR); PARK, Yang Hae, Seoul 140-713 (KR); SON, Hyun-Joo, Seoul 137-906 (KR); PARK, Eun-Ju, Suwon-si Gyeonggi-do 440-851 (KR); LEE, Bong-Yong, Seoul 137-060 (KR); NAMGUNG, Hojin, Seoul 139-784 (KR); PARK, Minseok, Suwon-si Gyeonggi-do 440-320 (KR); OH, Euichaul, Seongnam-si Gyeonggi-do 463-850 (KR)
(74) Representative: Gallagher, Kirk James
(86) International application number: PCT/KR2010/003197
(87) International publication number: WO 2010/134769

(57) **Abstract**

The present invention relates to a composition or a method for treating or preventing irritable bowel syndrome. The present invention uses *Atractylodes japonica* rhizome extract, preferably the ethanol water solution extract of *Atractylodes japonica* rhizome, more preferably the 35-65 v/v% ethanol water solution extract of *Atractylodes japonica* rhizome for treating or preventing irritable bowel syndrome. *Atractylodes japonica* rhizome extract shows a surprising effect in suppressing visceral hypersensitivity or improving defecation abnormality caused by stress or bowel irritability.

## Description

### [TECHNICAL FIELD]

The present invention relates to a pharmaceutical composition for treating or preventing irritable bowel syndrome, and medical-use thereof.

### [BACKGROUND ART]

Irritable bowel syndrome (IBS) is a chronic disease accompanied by abdominal pain; abdominal discomfort like chronic, repeated abdominal distention; and abnormality of evacuation like diarrhea, constipation, etc. in the absence of any detectable organic cause. The symptoms may worsen by mental factor or stress. IBS is classified as diarrhea-predominant IBS, constipation-predominant IBS or pain-predominant IBS, and the treatment of IBS is performed based on the symptoms. 30.8% of Korean IBS patients are diarrhea-predominant IBS, 24.6% are constipation-predominant IBS, and 44.6% have alternating stool pattern of diarrhea and constipation.

Medicine for treating IBS can be divided into both medicine for treating one symptom and medicine for relieving overall symptoms. Medicine for treating abdominal pain includes smooth muscle relaxant, antidepressant, opioid agonist, etc.; medicine for treating constipation-predominant IBS includes fiber preparation, alleviator, 5-HT4 agonist, etc.; and medicine for treating diarrhea-predominant IBS includes antidiarrheal, 5-HT3 antagonist, etc. However, the use of drugs related to 5-HT are very limited due to their side-effect, so there are few drugs for treating IBS until now. As a result, the treatment of IBS depends on only relieving IBS' symptoms, which are not satisfactory [TT. Ashburn et al., Nat. Rev. Drug Discov., 5(2), p 99-100, 2006; MJG Farthing, BMJ., 330, p 429-430, 2005; and MJG. Farthing, Best Pract. Res. Clin. Gastroenterol., 18(4), p 773-786,2004].

Meanwhile, neurokinin (NK) receptor is classified as subtypes of NK1, NK2 and NK3, and is a receptor binding with tachykinin family, which are neuropeptides acting on the central nervous system and the peripheral nervous system, such as substance P (SP), Neurokinin A, Neurokinin B and so on [S. Harrison et al., Int. J. Biochem. Cell Biol., 33: p 555-576, 2001]. This NK receptor is present in the central nervous system like brain's amygdala, hippocampus, hypothalamus, corpus striatum and spinal cord, or the peripheral nervous system like skin, inflammatory cell, digestive system, respiratory system, cardiovascular system, etc., and is closely connected with bowel movements and bowel irritability [JH. La et al., World J. Gastroenterol., 11(2), p 237-241, 2005; MS Kramer, Science, 281(5383) p 1624-1625. 1998; and G. J. Sanger., Br. J. Pharmacol., 141, p 1303-1312, 2004]. Recently, based on the physiological function of the NK receptor, antagonists of the NK receptor have been studied to develop new drugs for IBS [R. A. Duffy, Expert Opin. Emerg. Drugs, 9(1), 2004; M. Camilleri, Br. J. Pharmacol., 141, p 1237-1248, 2004; G. J. Sanger., Br. J. Pharmacol., 141, p 1303-1312, 2004; and A. Lecci et al., Br. J. Pharmacol., 141, p 1249-1263, 2004].

Until now, various herbal medicine (for example, *Inula helenium,* sclerotium of *Poria cocas, Angelica gigas, Paeonia japonica, Dioscorea japonica, Evodia officinalis,* trifoliate orange) are known as useful for relieving IBS, but they did not show a significant effect in CRD (Colorectal Distension) model, a representative evaluation model of IBS. IBS is clearly different from simple abdominal pain, simple diarrhea, or simple constipation because IBS is a chronic disease accompanied by diverse symptoms, for example, abdominal pain, abdominal distention, etc. with defecation abnormality like constipation, diarrhea and so on. Therefore, the various symptoms including pain-relieving effect, improvement of defecation abnormality, etc. should be evaluated together to determine the cure effect of any drug for IBS.

### [DISCLOSURE]

### [TECHNICAL PROBLEM]

Accordingly, the object of the present invention is to provide a composition useful for treating or preventing irritable bowel syndrome and an effective method for treating or preventing irritable bowel syndrome.

### [TECHNICAL SOLUTION]

To achieve the object, the present invention provides a pharmaceutical composition for treating or preventing irritable bowel syndrome (IBS), comprising *Atractylodes japonica* rhizome extract as an active agent. In addition, the present invention provides a method for treating or preventing irritable bowel syndrome, comprising administering to a subject in need thereof a therapeutically effective amount of *Atractylodes japonica* rhizome extract.

The inventors of the present invention confirmed that the extract of *Atractylodes japonica* rhizome suppresses the irritability of visceral pain and improves the defecation abnormality caused by stress or bowel irritability, and completed the present invention by verifying that the extract can be used usefully for treating or preventing IBS through those improvements. The effect of the extract according to the present invention is thought to be based on the fact that the extract inhibits NK receptor connected with sense delivery and bowel movability through the nociceptive pathway in the enteric nervous system, but the present invention is not limited to this kind of pharmacological mechanism.

Particularly, the present invention is based on the fact that the treatment effect of *Atractylodes japonica* rhizome extract on IBS is much superior to those of other plants in *Atractylodes* geuns, like *Atractylodes macrocephala, Atractylodes lancea* and so on.

*Atractylodes japonica* rhizome extract of the present invention can be made according to extracting methods well known in the art to which this invention pertains to. That is, the extract of the present invention can be made by drying the rhizomes in the shade and cutting them to pieces; extracting the cut rhizomes with 1 to 20 times of volume of extraction solvent; and then optionally concentrating (in reduced pressure), drying or purifying the resulting product. In more detail, in a process using solvent to extract active materials, (a) extraction can be performed at 50-100°C for 1-20 hours with a cooling condenser for blocking evaporation of extraction solvent, or (b) extraction can be performed at 5-37°C for 0.5-15 days by immersing the rhizomes in extraction solvent. The extract of the present invention can be made by stirring extraction method, reflux-cooling extraction method, cold water immersion method, sonication extraction method, supercritical extraction method and so on.

General extraction solvents, for example, C₁₋₄ lower alcohol or their aqueous solution; polyhydric alcohol like glycerin, butylene glycol, propylene glycol, etc.; hydrocarbon solvent like methyl acetate, ethyl acetate, acetone, benzene, hexane, diethyl ether, dichloromethane, etc.; or their mixture solvent, may be used for preparing the extract of the present invention. However, ethanol aqueous solution (for example, 5-95 (v/v) % ethanol water solution) is preferable as extraction solvent when considering the efficacy of treating IBS, 20-80 (v/v) % of ethanol aqueous solution is more preferable, and 35-65 (v/v) % of ethanol aqueous solution is most preferable. The extract of *Atractylodes japonica* rhizome made with these extraction solvents shows much better medicinal effect for IBS than extracts made with other solvents or other ratios of ethanol aqueous solution.

*A trachjlodes japonica* rhizome extract is preferably present at a level of from 1 % to 90% by weight, and more preferably from 10% to 60% by weight of the composition of the present invention.

The composition for treating or preventing irritable bowel syndrome according to the present invention can be formulated into forms of medicine and functional food. These medicine and functional food can comprise pharmaceutically acceptable excipient or additives. The composition according to the present invention may be administered alone or with any convenient carrier, diluent, etc. and the formulation for administration may be single-dose unit or multiple-dose unit.

The medicine and functional food comprising the composition of the present invention may be formulated in a solid or liquid form. The solid formulation includes, but is not limited to, a powder, a granule, a tablet, a capsule, a suppository, etc. Also, the solid formulation may further include, but is not limited to, a diluent, a flavoring agent, a binder, a preservative, a disintegrating agent, a lubricant, a filler, etc. The liquid formulation includes, but is not limited to, a solution such as water solution and propylene glycol solution, a suspension, an emulsion, etc., and may be prepared by adding suitable additives such as a coloring agent, a flavoring agent, a stabilizer, a thickener, etc.

For example, a powder can be made by simply mixing *Atractylodes japonica* rhizome extract of the present invention and pharmaceutically acceptable excipient like lactose, starch, microcrystalline cellulose and so on. A granule can be prepared as follows: mixing *Atractylodes japonica* rhizome extract, a pharmaceutically acceptable diluent and a pharmaceutically acceptable binder such as polyvinylpyrrolidone, hydroxypropylcellulose, etc; and wet-granulating with adequate solvent like water, ethanol, isopropanol, etc, or direct-compressing by compressing power. In addition, a tablet can be made by mixing said granule with a pharmaceutically acceptable lubricant such as magnesium stearate, and tabletting the mixture.

The composition of the present invention may be administered in forms of, but not limited to, oral formulation, injectable formulation (for example, intramuscular, intraperitoneal, intravenous, infusion, subcutaneous, implant), inhalable, intranasal, vaginal, rectal, sublingual, transdermal, topical, etc. depending on the disorders to be treated and the patient's conditions. The composition of the present invention may be formulated in a suitable dosage unit comprising a pharmaceutically acceptable and non-toxic carrier, additive and/or vehicle, which all are generally used in the art, depending on the routes to be administered. A depot type of formulation being able to continuously release drug for desirable time also is included in the scope of the present invention.

For achieving the object of treating or preventing irritable bowel syndrome, *Atractylodes japonica* rhizome extract of the present invention may be administered daily at a dose of approximately 10 mg/kg to approximately 2,400 g/kg, preferably approximately 100 mg/kg to approximately 1,200 mg/kg. However, the dosage may be varied according to the patient's conditions (age, sex, body weight, etc.), the severity of patients in need thereof, the used effective components, diets, etc. The composition of the present invention may be administered once a day or several times a day in divided doses, if necessary.

IBS that can be treated, relaxed or prevented by the composition of the present invention is at least any one selected from diarrhea-predominant IBS, constipation-predominant IBS, or pain-predominant IBS.

In addition, the present invention provides a method for inhibiting NK2 (Neurokinin2) receptor, comprising administering to a mammal including human in need thereof *Atractylodes japonica* rhizome extract. The present invention also provides a method for inhibiting NK2 (Neurokinin2) receptor, comprising contacting *Atractylodes japonica* rhizome extract with cells expressing NK2 receptor.

### [ADVANTAGEOUS EFFECTS]

The present invention provides a composition for treating or preventing IBS, comprising *Atractylodes japonica* rhizome extract as an effective agent. The present invention also provides a method for treating or preventing IBS, comprising administering to a subject in need thereof a therapeutically effective amount of *Atracrylodes japonica* rhizome extract.

### [DESCRIPTION OF DRAWINGS]

Figure 1 is HPLC chromatography results of *Atractylodes japonica* rhizome extract.
Figure 2 is thin layer chromatography (TLC) results of *Atractylodes japonica* rhizome extract.
Figure 3 is a graph showing efficacy of *Atractylodes japonica* rhizome extract in the CRD model.
Figure 4 is a graph showing efficacy of *Atractylodes japonica* rhizome extract in the restraint stress-induced fecal pellet output model.
Figure 5 is a graph comparatively showing efficacy of *Atractylodes macrocephala* rhizome extract and *Atractylodes japonica* rhizome extract in the CRD model.
Figure 6 is a graph comparatively showing efficacy of *Atractylodes lancea* rhizome extract and *Atractylodes japonica* rhizome extract in the CRD model.

### [MODES FOR CARRYING OUT THE INVENTION]

Hereinafter, the present invention is described in considerable detail to help those skilled in the art understand the present invention. However, the following examples are offered by way of illustration and are not intended to limit the scope of the invention. It is apparent that various changes may be made without departing from the spirit and scope of the invention or sacrificing all of its material advantages.

### <Preparation of Atractylodes japonica Rhizome Extract >

Ethanol-water solution extract of *Atractylodes japonica* rhizome was prepared. 100 g of the rhizome was dried in the shade and cut to pieces. 0.7 liter of 30, 50, 70 or 90 (v/v) % ethanol aqueous solution was added, and stirred for 4 hours during when 2 times reflux condenser extraction were performed. The extract was filtered, concentrated and dried. The final product was used in the following experiments.

Water extraction of *Atractylodes japonica* rhizome was prepared as follows: 100 g of the rhizome was added into an Herb Extractor (Daewoong Co., Model No. DWP5000M) and 1.5 liter of purified water was added to them. Then the first extraction switch was on for 150 minutes of the first extraction. After that, the same amount of water was again added, and the second extraction switch was on for 120 minutes of the second extraction. The extract was then filtered, concentrated and dried. The final product was used in the following experiments.

### <Evaluation on Difference of Extracted Ingredients>

### Evaluation using HPLC

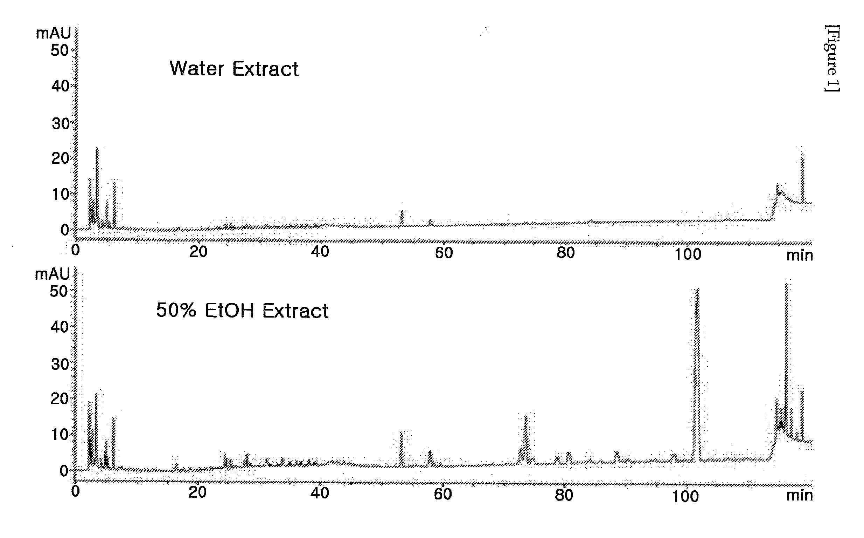

Figure 1 is HPLC chromatogram results of water extract and 50% ethanol water solution extract of *Atractylodes japonica* rhizome [System: Agilent 1200 Series; Column: C18 column (250×4.6mm ID, S-5µm, 12nm); Detection wavelength: 210nm]. Peaks after 60 minutes of Rt were not found in water extract unlike 50% ethanol solution extract.

### Evaluation using TLC

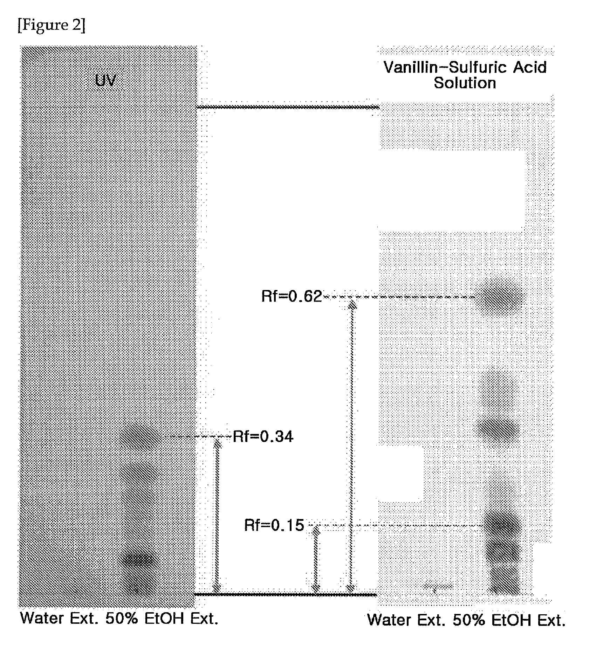

Thin layer chromatography (TLC) was performed for confirming the difference of extract ingredients shown in figure 1. Normal hexane was added to each extract to make a 100 mg/mL of concentration, and then sonicated for 15 minutes. Insoluble materials were removed by filtration, and the filtered solutions were used as sample solutions. The samples were added dropwise to silica gel plate (Silica gel 60F 254, Merck), and then developed by using 10:1 (v/v) mixture of normal hexane and ethyl acetate as a developing solvent. Then, the color and position of spots occurred in the dried silica gel plate was identified by ultraviolet rays (about 254 nm) and compared with each other. For naked eye's observation, vanillin-sulfuric acid solution (5% sulfuric acid ethanol solution, 1% vanillin ethanol solution) was sprayed and dried at 110°C for about 5-10 minutes. Then, spots were color-reacted and observed. The results were shown in figure 2.

As shown in figure 2, no specific spots except for weak spots near the baseline (Rf=0) were observed in both of the UV and color reagents tests when the water extract is used, but when 50% ethanol solution extract was used, diverse spots were observed at about Rf 0∼0.34 in the UV (254nm) test, and navy or purple spots were observed at about Rf 0.15 and pink spots were also observed at about Rf 0.62 in the color reagents test. These results show that the kind of the extraction solvent causes big difference of extracted ingredients when preparing *Atractylodes japonica* rhizome extract.

### <Evaluation on Suppressing Effect of Abdominal Pain in CRD model>

To evaluate the suppressing effect of *Atractylodes japonica* rhizome extracts on the abdominal irritability, Colorectal Distension (CRD) test that has been often used for evaluating drugs for IBS was used as follows [JH. La et al., World J. Gastroenterol., Dec., 9(12): p 2791-2795, 2003].

250-300g of Sprague-Dawley male rats (Charles River) were used. Two rats per one cage were reared in a room of 25°C, humidity 50%, and day-night 12:12 hours of cycle. Rats could freely drink water and eat feed, and were adapted to the room condition for 5 days. Then colonitis was caused in those rats. Feed was stopped 24 hours before causing colonitis, and ether was used for breathing anesthesia. Rubber catheter (PE 50) was inserted from the anus up to 8cm through rectum.

1ml of 3.5% acetic acid (acetic acid in 0.9% saline) was administered though the catheter into the lumen of the colon, and then the anus was tied to block the leakage of the solution. After 30 seconds, 1ml of 0.9% saline was administered through the same catheter into the lumen of the colon to wash away the acetic solution.

A 2 cm length of rubber balloon was inserted into the rectum of each rat, and 37°C water was filled in the balloon, by stages, from 0.1ml to 1.0ml. The appearing pain reactions of rats were recorded. The specific behaviors of CRD test animals were recorded with AWR score used for indirect quantitative analysis of AWR (abdominal withdrawal reflex), and predetermined scores were given on each behavior and AWR score is used for identifying the abdominal pain reaction. AWR scores [E. D. Al-Chaer et al., Gastroenterology, Nov., 119(5), p 1276-1285. 2000] according to table 1 were recorded.

**[Table 1]**

| AWR Score | Specific Behavior |
|---|---|
| 0 | No behavioral response to distension |
| 1 | Brief head movements followed by immobility |
| 2 | Contraction of abdominal muscle without lifting of abdomen |
| 3 | Lifting of abdomen |
| 4 | Body arching and lifting of pelvic structure |

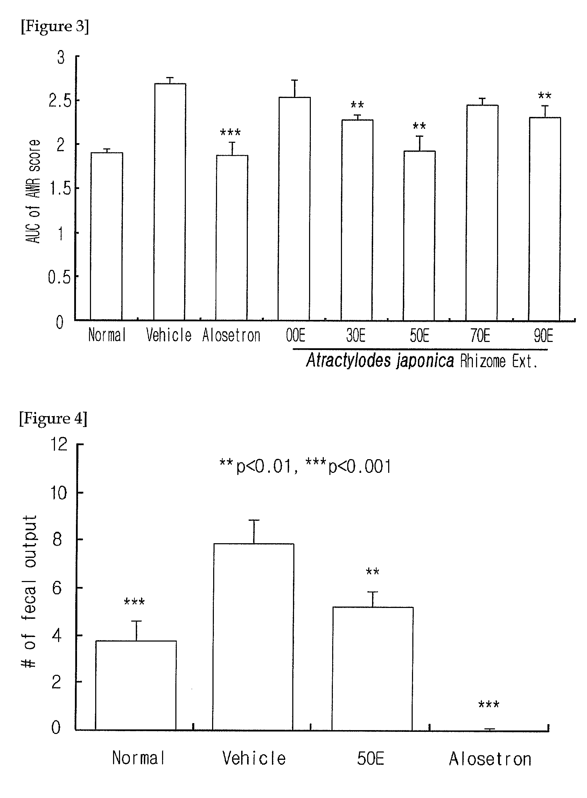

The presence of visceral hypersensitivity was checked in CRD rats 7 days after causing colonitis. Through this checking, model animals having symptoms like IBS were selected [JH. La et al., World J. Gastroenterol., Dec., 9(12): p 2791-2795, 2003]. Each *Atractylodes japonica* rhizome extract was dissolved in 0.5% carboxymethylcellulose (CMC) water solution, and 200 mg/kg of extract was orally administered. 20 mg/kg of positive control, alosetron HCL (Jiangyin Yongda Chemical Co., Ltd.) was orally administered. Then, rats were stabilized for about 1 hour, and then CRD tests were performed to record AWR score. AWR score according to distension volume (ml) and its AUC (area under the curve) were calculated to quantify results of reaction in vehicle-administered group, positive control and extract-administered group. Student's t-test (p<0.01 (**) or p<0.001 (***)) was used for statistical approach, and significance compared with the vehicle-administered group was checked. The results were shown in figure 3 (mean±S.E., n≥5).

In figure 3, "Normal" means normal rats without causing colonitis, "Vehicle" means orally only vehicle-administered colonitis group, "Alosetron" means orally 20mg/kg of alosetron-administered colonitis group (positive control), "00E" means orally 200mg/kg of water extract-administered colonitis group, and "30E", "50E", "70E" and "90E" mean, respectively, 200mg/kg of 30%, 50%, 70% and 90% ethanol extract-administered colonitis group.

As shown in figure 3, 50% ethanol aqueous solution showed a strong suppressing effect against the visceral pain occurring in the visceral hypersensitivity caused by colonitis, which efficacy was about equal to that shown by 20mg/kg of alosetron, the positive control. Meanwhile, water extract showed little suppressing efficacy. 30% ethanol extract and 90% ethanol extract showed more or less suppressing activity, but the magnitude of the activity was very weaker than that of 50% ethanol solution extract. Therefore, the *Atrachjlodes japonica* rhizome extract made by 35 to 65% ethanol solution is the most preferable.

### <Efficacy Evaluation in Restraint Stress-induced Fecal Pellet Output Model>

50% Ethanol extract that showed the best suppressing effect against the colon hypersensitivity in the above experiment was evaluated with the restraint stress-induced fecal pellet output model often used for evaluating the treatment effect of IBS [S. Kobayashi et al., Jpn. J. Pharmcaol., 86, p 281-288, 2001].

250-300g of Sprague-Dawley male rats (Charles River) were used. Two rats per one cage were reared in a room of 25°C, humidity 50%, and day-night 12:12 hours of cycle. Rats could freely drink water and eat feed, and were adapted to the room condition for 5 days. Then the test was performed.

On the day of the experiment, the restraint-induced fecal pellet output of rats was evaluated with a restraint cage. 50% Ethanol solution extract was dissolved in 0.5% CMC water solution, and 300 mg/kg of extract was orally administered. Alosetron, a positive control, was orally administered at a concentration of 20mg/kg. Then, test animals were fit into the restraint cage. Much attention was paid not to stress the animals. The restraint cage blocked the animals from moving, which caused the restraint stress, which consequently caused the starting of defecation.

The appearance and number of feces was evaluated every 60 minutes for 4 hours, and the results were shown in figure 4. Student's t-test was used for statistical approach, and significance was checked at a level of p<0.01 (**) or p<0.001 (***).

300 mg/kg of 50% ethanol extract reduced the number of fecal pellet output caused by the restraint, from 7.9 times of the vehicle group to 5.2 times. This result means that the extract of the present invention can improve the fecal output abnormality caused by the restraint stress. From this result, *Atractylodes japonica* rhizome extract of the present invention is confirmed to have an improving activity against the fecal output abnormality caused by the restraint stress.

### <Evaluation of Activity Inhibiting Neurokinin Receptor>

Cell line (U-373MG glioma cell line) over-expressing neurokinin receptor was purchased from Korean Cell Line Bank. The cell line was incubated with 5% CO₂ in a medium (90% of RPMI 1640, 10% of fetal bovine serum, 100 IU/ml of penicillin, 100 µg/ml of streptomycin, 2mM of L-glutamine, and 1.0mM of sodium pyruvate), and was sub-cultured every 6-8 days with 0.25% trypsin solution.

Cells were diluted to have various cell numbers, and were seeded onto a 24-well plate. After 48 hours, substance P (SP) connected with a probe was added in various concentrations. After some time for reaction, the concentration of fluorescence probe-containing SP (Oregon Green488 conjugate, Molecular Probes, Eugene, OR) bound to the receptor was measured with a fluorophotometer according to the number of cells and the concentrations, through which the ideal condition was established [VJ. Bennett et al., BMC Chem. Biol., 1 : 1, 2001]. Non-peptide antagonist, L-733060 (estimated affinity = 0.8nM), which selectively binds to the receptor was used as a control [R. Bang et al., J. Pharmacol. Exp. Ther., 305, p 31-39, 2003].

50% Ethanol aqueous solution extract of *Atractylodes japonica* was solved in DMSO (the final concentration is within 0.1 %) and diluted with distilled water. Then germs of the solution was removed by filtering the solution with a membrane (millipore membrane, 0.22 µm, Millex-GV, U.S.A.), and the aseptic solution was administered at concentrations of 10µg/ml and 50µg/ml. After control cells were treated with vehicle (0.1% DMSO), water was added instead of SP. In the group treated with only SP, cells was pre-treated with vehicle (0.1 % DMSO) and then treated with 50nM of SP. In the group treated with the extract, cells were treated with the extract and, after 1 hour, treated with 50nM of SP. In the group treated with L-733060, cells was treated with 50nM of L-733060 and then treated with 50nM of SP. Reaction was performed for 1 hour in an incubator, and cells were washed with serum free media. Then, cells were dissolved in 5% Triton X-100, and their fluorescence (Ex 485/Em 528) was measured with black, opaque 96-well plate. The number of sample per each test was two (n=2), and the same test was repeated three times. The results are shown in table 2 below.

**[Table 2]**

| Sample | Conc. | Relative Inhibitory Activity (%) |
|---|---|---|
| Control | - | 100 |
| Vehicle | - | 0 |
| L-733060 | 50 nM | 72 ± 8** |
| 50% EtOH Extract | 10 µg/ml | 33 ± 1* |
| | 50 µg/ml | 45 ± 8* |

As shown in the table 2, 10 µg/ml and 50 µg/ml of 50% ethanol extract inhibited the neurokinin receptor by 33% and 45%, respectively. From these results, the effect of the *Atractylodes japonica* rhizome extract according to the present invention is thought to be based on the fact that the extract inhibits NK receptor connected with sense delivery and bowel movability through the nociceptive pathway and consequently improves visceral hypersensitivity and defecation abnormality, but the present invention is not limited to this kind of pharmacological mechanism.

### <Comparative Evaluation of Atractylodes macrocephala and Atractylodes japonica>

The IBS-treating effect of the same genus plants, *Atractylodes macrocephala* and *Atractylodes japonica* was comparatively evaluated. The rhizome of *Atractylodes japonica* has much smaller cross-section than the rhizome of *Atractylodes macrocephala,* so that the two rhizomes can be easily discriminated with a naked eye.

100 g of each rhizome was dried in the shade and cut to pieces. 0.7 Liter of 50% ethanol aqueous solution was added to each cut rhizome and then well stirred for 4 hours, during when 2 times reflux condenser extraction were performed. The resulting extract was filtered, concentrated and dried. The final product was used in the following experiments.

### UPLC Comparative Evaluation

The difference of ingredients in *Atractylodes japonica* and *Atractylodes macrocephala* 50% ethanol water solution extract was measured by UPLC chromatogram [System: ACQUITY UPLC; Column: ACQUITY UPLC HSS T3 1.8um, 2.1×150mm; Detection wavelength: 220nm]. Results showed that the extracted ingredients were different from each other.

### Comparative Evaluation on Suppressing Effect of Visceral Pain in CRD (Colorectal Distension) Model

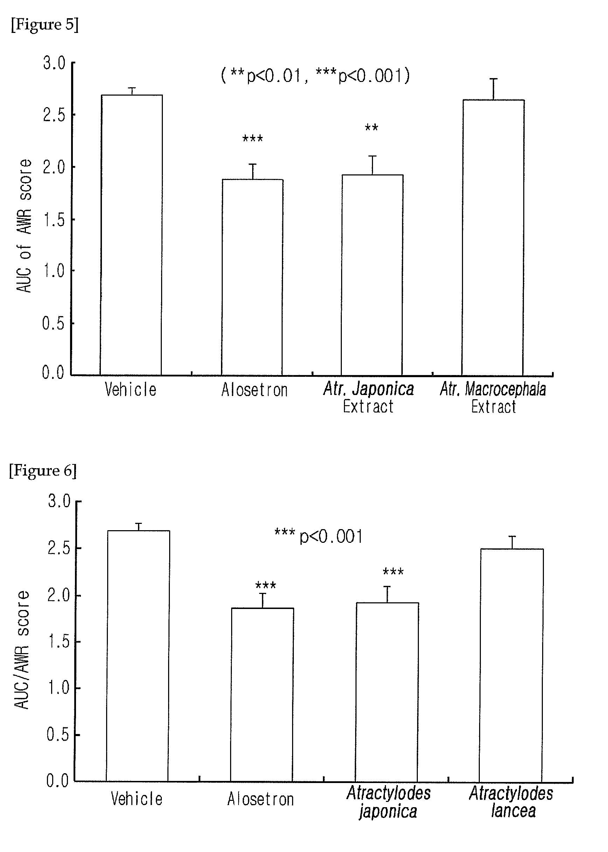

The IBS-treating effect of *Atractylodes japonica* rhizome 50% ethanol extract (200mg/kg) and *Atractylodes macrocephala* rhizome 50% ethanol extract (200mg/kg) was comparatively evaluated in the CRD model. The results are shown in figure 5.

As shown in figure 5, *Atractylodes japonica* rhizome extract showed a significant effect of suppressing the visceral pain of the visceral irritability state in comparison to the vehicle, while *Atractylodes macrocephala* rhizome extract did not show a significant difference.

### Comparative Evaluation on Activity to Inhibit Neurokinin (NK) 2 Receptor

The NK2 receptor-inhibiting effect of *Atractylodes japonica* rhizome 50% ethanol extract and *Atractylodes macrocephala* rhizome 50% ethanol extract was comparatively evaluated at a concentration of 100 µg/ml. The test was performed at MDS Pharma Services, and the results are shown in table 3 below.

**[Table 3]**

| 100 µg/ml | *Atractylodes Japonica* Extract | *Atractylodes macrocephala* Extract |
|---|---|---|
| NK2 Receptor Inhibitory Activity | 64 % | 19 % |

As shown in table 3, *Atractylodes macrocephala* rhizome extract showed little inhibiting effect, but *Atractylodes japonica* rhizome extract showed a good inhibiting effect against the NK2 receptor by over 50%.

### <Comparative Evaluation of Atractylodes lancea and Atractylodes japonica>

The IBS-treating effect of the same genus plants, *Atractylodes lancea* and *Atractylodes japonica* was comparatively evaluated. 100 g of each rhizome was dried in the shade and cut to pieces. 0.7 Liter of 50% ethanol aqueous solution was added to each cut rhizome and then well stirred for 4 hours, during when 2 times reflux condenser extraction were performed. The resulting extract was filtered, concentrated and dried. The final product was used in the following experiments.

The visceral pain-suppressing effect of each extract was comparatively evaluated in the CRD (Colorectal Distension) model. *Atractylodes japonica* rhizome 50% ethanol aqueous solution extract and *Atractylodes lancea* rhizome 50% ethanol aqueous solution extract was administered at a dose of 200 mg/kg, 1 hour before AWR score is checked. Alosetron was orally administered at a dose of 20 mg/kg. The results are shown in figure 6.

As shown in figure 6, *Atractylodes japonica* rhizome extract showed a significant effect of suppressing the visceral hypersensitivity in comparison to the vehicle, while *Atractylodes lancea* rhizome extract did not show a significant difference.

### <Formulation Examples>

Hereinafter, the formulation of the composition comprising the extract of the present invention is illustratively disclosed, but the present invention is not limited to these examples.

### Preparation of Powder

Ingredients: The extract of the present invention 20 mg; lactose 100 mg; and talc 10 mg.

Powder was prepared by mixing the above ingredients and filling the mixture into an airtight bag.

### Preparation of Tablet

Ingredients: The extract of the present invention 10 mg; corn starch 100 mg; lactose 100 mg; and magnesium stearate 2 mg.

Tablets were made by mixing the above ingredients and tabletting the mixture according to a method well known in the art.

### Preparation of Capsule

Ingredients: The extract of the present invention 10 mg; crystalline cellulose 3 mg; lactose 14.8 mg; and magnesium stearate 0.2 mg.

Capsules were prepared by mixing the above ingredients and filling the mixture in gelatin capsules according to a method well known in the art.

### Preparation of Injectable Solution

Ingredients: The extract of the present invention 10 mg; mannitol 180 mg; sterile water for injection 2974 mg; and Na2HPO₄·12H₂O 26 mg.

Injectable solution was prepared according to a well known method, and the above ingredients was included in 1 ample (2Mℓ).

### Preparation of Solution

Ingredients: The extract of the present invention 20 mg; isomerized glucose syrup 10 g; mannitol 5 g; and water q.s.

According to a well known method for making a solution, each ingredient was dissolved in water, and a little of lemon flavor was added to the solution and mixed again. Water was added to make the total volume 100 Mℓ, and then the resultant was filled in a brown bottle and was sterilized.

### Preparation of Healthy Food

Ingredients: The extract of the present invention 1,000 mg; vitamin mixture q.s.; vitamin A acetate 70 µg; vitamin E 1.0 mg; vitamin B1 0.13 mg; vitamin B2 0.15 mg; vitamin B6 0.5 mg; vitamin B12 0.2 µg; vitamin C 10 mg; biotin 10 µg; nicotine acid amide 1.7 mg; folic acid 50 µg; Calcium pantothenate 0.5 mg; mineral mixture q.s.; ferrous sulfate 1.75 mg; Zinc oxide 0.82 mg; Magnesium carbonate 25.3 mg; monopotassium phosphate 15 mg; dipotassium phosphate 55 mg; potassium citrate 90 mg; calcium carbonate 100 mg; and magnesium chloride 24.8 mg.

According to a well known method for making a healthy food, the above ingredients were mixed and prepared as granules. The granules may be formulated to tablet or capsule according to a well known method.

### Preparation of Healthy Drink

Ingredients: The extract of the present invention 1,000 mg; citric acid 1,000 mg; oligosaccharide 100 g; concentrated plum solution 2 g; taurin 1 g; and purified water q.s. (total 900 Mℓ).

According to a well known method for making a healthy drink, the above ingredients were mixed and heated at 85°C for about 1 hour. Then, the resulting solution was filtered, filled in a 2ℓ of sterile container, sealed and sterilized to make the healthy drink.

## Claims

1. A composition for treating or preventing irritable bowel syndrome, comprising the extract of *Atractylodes japonica* rhizome as an active agent.

2. The composition of claim 1, wherein the extract is the extract made with an ethanol water solution.

3. The composition of claim 2, wherein the ethanol water solution is 35 to 65 (v/v) % of the ethanol water solution.

4. A method for treating or preventing irritable bowel syndrome, comprising administering a therapeutically effective amount of *Atractylodes japonica* rhizome extract to a subject in need thereof.

5. The method of claim 4, wherein the extract is the extract made with an ethanol water solution.

6. The method of claim 5, wherein the ethanol water solution is 35 to 65 (v/v) % of the ethanol water solution.

7. A medical use of *Atractylodes japonica* rhizome extract for treating or preventing irritable bowel syndrome.

8. The medical use of claim 7, wherein the extract is the extract made with an ethanol water solution.

9. The medical use of claim 8, wherein the ethanol water solution is 35 to 65 (v/v) % of the ethanol water solution.

10. A method for inhibiting NK2 receptor, comprising administering extract of *Atractylodes japonica* rhizome to a subject in need thereof.
